# EUROPÄISCHE PATENTSCHRIFT

(11) **EP 0 994 188 B1**
(45) Veröffentlichungstag und Bekanntmachung des Hinweises auf die Patenterteilung: **07.01.2004**
(21) Anmeldenummer: 98119077.0
(22) Anmeldetag: 09.10.1998
(51) Int. Cl.: C12N 15/12, C07K 14/48

(54) **Verfahren zur Gewinnung von aktivem Beta-NGF**
Method for obtaining active beta-NGF
Procédé d'obtention du NGF-bêta biologiquement actif

(43) Veröffentlichungstag der Anmeldung: 19.04.2000
(73) Patentinhaber: Scil proteins GmbH, 06120 Halle/Saale (DE)
(72) Erfinder: RATTENHOLL, Anke, D-06120 Halle/Saale (DE); GROSSMANN, Adelbert, Dr., D-82436Eglfing (DE); SCHWARZ, Elisabeth, D-06114 Halle/Saale (DE); RUDOLPH, Rainer, Dr., D-06114 Halle/Saale (DE)
(74) Vertreter: Behnisch, Werner, Dr.

(56) Entgegenhaltungen:
- EP-A- 0 544 293
- EP-A- 0 786 520
- WO-A-96/08562
- WO-A-97/28272
- SUTER U. ET AL.: "Two conserved domains in the NGF propeptide are necessary and sufficient for the biosynthesis of correctly processed and biologically active NGF" THE EMBO JOURNAL, Bd. 10, Nr. 9, 1991, Seiten 2395-2400, XP002095460

## Beschreibung

Die vorliegende Erfindung betrifft ein Verfahren zur Gewinnung von β-NGF durch Naturierung von denaturiertem, inaktiven proNGF und Abspaltung der Prosequenz.

Nervenwachstumsfaktor (β-NGF) ist ein neurotropher Faktor, der für das Wachstum und das Überleben von sympathischen und sensorischen Neuronen notwendig ist (R. Levi-Montalcini, Science 237, 1154 (1987); H. Thoenen und Y.-A. Barde, Physiol. Rev. 60, 1284 (1980); B. A. Yankner und E. M. Shooter, Annu. Rev. Biochem. 51, 845 (1982)). β-NGF unterstützt außerdem Wachstum, Differenzierung und Vitalität cholinerger Neuronen des zentralen Nervensystems (F.J. Hefti, J. Neurobiol. 25, 1418 (1994)). Mögliche therapeutische Indikationen für rekombinanten menschlichen Nervenwachstumsfaktor beinhalten periphere sensorische Neuropathien, z.B. bei Diabetes oder als mögliche Nebenwirkung bei der AIDS-Therapie. Weitere Indikationen für rh-β-NGF stellen zentrale Neuropathien, z.B. die Alzheimersche Krankheit dar. Der Gedächtnisverlust wird hier durch den Verlust cholinerger Neuronen hervorgerufen.

Menschlicher β-NGF wird als Präpro-Protein translatiert. Dieses besteht aus 241 Aminosäuren. Das Präpeptid (18 Aminosäuren) wird bei der Translokation ins endoplasmatische Retikulum (ER) abgespalten, das entstandene Proprotein wird anschließend N- und C-terminal prozessiert (Abspaltung der Prosequenz (103 Aminosäuren) und der letzten beiden Aminosäuren). Reifer humaner NGF besteht folglich aus 118 Aminosäuren. Er ist homolog zum Maus-β-NGF und unterscheidet sich von diesem Protein lediglich durch 12 Aminosäureaustausche. Für die Durchführung klinischer Studien oder einen möglichen Einsatz als Therapeutikum müssen große Mengen homogenen β-NGFs verfügbar sein. Eine natürliche Quelle größerer Mengen dieses Faktors sind die Submaxillardrüsen männlicher Mäuse. Diese Präparationen sind jedoch heterogene Gemische verschiedener Dimerer und daher für einen therapeutischen Einsatz ungeeignet. Außerdem ist es wünschenswert, Patienten die humane Form des Proteins zu verabreichen. Neurotrophe Faktoren sind aber nur in verschwindend geringen Konzentrationen im menschlichen Gewebe vorhanden.

Für den Einsatz von β-NGF als Therapeutikum kommt deshalb nur die rekombinante Herstellung in Betracht. Hier bieten sich zwei Möglichkeiten an: die rekombinante Expression entweder in Zellkultur oder in Bakterien. Eukaryotische Zellexpressionssysteme liefern jedoch nur sehr geringe Proteinmengen und sind verhältnismäßig teuer (J. Bamett et al., J. Neurochem. 57, 1052 (1991); C. H. Schmelzer et al., J. Neurochem. 59, 1675 (1992) ; R. H. Edwards und W. J. Rutter, United States Patent No. 5,683,894).

Im Gegensatz dazu werden mit prokaryotischen Expressionssystemen große Mengen des gewünschten Proteins erhalten. Bakterien sind jedoch im Gegensatz zu den eukaryotischen Expressionssystemen nicht in der Lage, die Vorläuferproteine korrekt zu prozessieren. Die Produktion rekombinanten β-NGFs führt in Bakterien, wie häufig bei der Expression von rekombinanten Säugergenen, zum biologisch inaktiven Translationsprodukt, welches dann in aggregierter Form in der Zelle abgelagert wird (sog. "Inclusion Bodies" (IBs)).

Die Naturierung von reifem β-NGF aus derartigen Inclusion Bodies gelingt jedoch nur bei sehr geringen Proteinkonzentrationen (unter 10 µg/ml) und bei sehr geringen Ausbeuten (bis ca. 10 %). Solche Verfahren sind beispielsweise beschrieben in der EP-A 0 544 293, US-Patent 5,606,031, US-Patent 5,235,043 und WO 97/47735. Eine Naturierung über eine Sulfitolyse von neurotrophen Faktoren der NGF/BDNF-Familie wird in der WO 95/30686 beschrieben.

In der WO 97/47735 wird ein verbessertes Verfahren zur Naturierung von Proteinen beschrieben. In diesem Verfahren wird das inaktive Protein mit einer Lösung eines Denaturierungsmittels in einer denaturierenden Konzentration in Gegenwart einer niedermolekularen Substanz, welche Thiolgruppen enthält, gelöst. Das gelöste Protein wird anschließend aus der stark denaturierenden Lösung in eine nicht oder nur schwach denaturierende Lösung überführt, in welcher es eine biologisch aktive Konformation annimmt, wobei die Disulfidbindungen mit der Thiolkomponente gelöst werden und im Protein intramolekular in der Weise neu gebildet werden, daß das Protein eine Konformation einnimmt, die biologische Aktivität aufweist. Mit einem solchen verbesserten Verfahren kann für β-NGF eine Naturierungsausbeute von ca.10% erhalten werden.

Aufgabe der vorliegenden Erfindung ist es, ein verbessertes Verfahren zur Gewinnung von β-NGF zur Verfügung zu stellen, welches einfach ist und aktiven NGF in hohen Ausbeuten liefert.

Die Aufgabe wird gelöst durch ein Verfahren zur Gewinnung eines biologisch aktiven β-NGF durch Naturierung der in inaktiver schwerlöslicher Form vorliegenden Proform, wobei die Proform (proNGF) vorzugsweise erhältlich ist in Form von Inclusion Bodies nach rekombinanter Herstellung in Prokaryonten, wobei das Verfahren dadurch gekennzeichnet ist, daß der proNGF in seiner inaktiven schwer löslichen Form mit einer Lösung eines Denaturierungsmittels in einer denaturierenden Konzentration gelöst wird, anschließend unter Erhalt der Löslichkeit in eine nicht oder schwach denaturierende Lösung überführt wird und dabei - gelöster denaturierter proNGF eine biologisch aktive Konformation annimmt, die durch die im natürlichen β-NGF vorliegenden Disulfidbrücken bestimmt ist und anschließend die Prosequenz abgespalten wird, wobei aktiver β-NGF erhalten wird, der isoliert werden kann.

Es hat sich überraschenderweise gezeigt, daß die Prosequenz bei der Naturierung von inaktivem β-NGF in vitro einen wesentlichen und positiven Einfluß auf das Naturierungsverfahren hat und es erfindungsgemäß möglich ist, die Renaturierung auf einfachste Weise durchzuführen und dabei dennoch bisher nicht gekannte und für möglich gehaltene Ausbeuten an naturiertem, aktiven β-NGF zu erhalten.

Unter "proNGF" ist β-NGF zu verstehen, welcher am N-Terminus mit seiner Prosequenz verknüpft ist. Erfindungsgemäß wird als Prosequenz die gesamte Prosequenz (R. H. Edwards und W. J. Rutter, US Patent 5,683,894; A. Ullrich et al. Nature 303, 821(1983), 821; SWISS-PROT Protein Sequence Database No. P01138) verwendet. U. Suter et al. (EMBO J. 10, 2395 (1991)) haben die in vivo-Funktion des Propeptids von murinem β-NGF hinsichtlich der korrekten Sekretion in einem COS-7-Zellkultursystem näher untersucht. Dazu wurde die Prosequenz in fünf Bereiche eingeteilt. Es wurden Mutanten hergestellt, in denen ein oder mehrere dieser Sequenzen deletiert wurden. Dabei wurde gefunden, daß die Sequenzbereiche mit den Aminosäuren -52 bis -26 ("Domäne I") sowie -6 bis -1 ("Domäne II") für die Expression und Sekretion von biologisch aktivem β-NGF essentiell sind. Domäne I ist wichtig für die Expression, während Domäne II die korrekte proteolytische Prozessierung bewirkt. Es hat sich überraschenderweise gezeigt, daß proNGF in analoger Weise wie β-NGF eine Aktivität in vivo zeigt. proNGF kann damit ebenfalls als Therapeutikum verwendet werden.

Inaktiver, schwer löslicher proNGF entsteht bei der Überexpression des Proteins im Cytosol von Prokaryoten. Rekombinant hergestellter proNGF verbleibt dabei in unlöslicher und aggregierter Form im Cytoplasma. Derartige Aggregate von Proteinen, deren Isolierung und Reinigung sind beispielsweise in F. A. Marston, Biochem. J. 240 (1986) 1-12 beschrieben. Zur Isolierung dieser inaktiven Proteinaggregate (Inclusion Bodies) werden die prokaryotischen Zellen nach der Fermentation aufgeschlossen.

Der Zellaufschluß kann durch übliche Methoden durchgeführt werden, z.B. mittels Ultraschall, Hochdruckdispersion oder Lysozym (R. Rudolph et al.: Folding proteins. In: T.E. Creighton (ed.): Protein Function: A Practical Approach. Oxford University Press, pp. 57-99 (1997)). Er wird vorzugsweise in einer zur Einstellung eines neutralen bis schwach sauren pH-Werts geeigneten Pufferlösung als Suspensionsmedium durchgeführt, wie z.B. 0.1 mol/l Tris-HCl. Nach Zellaufschluß werden die unlöslichen Bestandteile (Inclusion Bodies) in beliebiger Weise abgetrennt, vorzugsweise durch Abzentrifugieren oder Filtration nach einem oder mehreren Waschschritten, mit Agentien, die IBs nicht zerstören, fremde Zellproteine jedoch möglichst lösen, z.B. Wasser oder Phosphatpuffer, ggf. unter Zusatz milder Detergentien, wie Brij®. Anschließend wird der unlösliche Anteil (Pellet) dem erfindungsgemäßen Verfahren zur Solubilisierung und Naturierung unterworfen.

Als Denaturierungsmittel wird zweckmäßig ein für die Solubilisierung von Inclusion Body Proteinen üblicherweise verwendetes Denaturierungsmittel eingesetzt. Bevorzugt werden Guanidiniumhydrochlorid und andere Guanidiniumsalze, wie z.B. Thiocyanat sowie Harnstoff oder dessen Derivate, verwendet. Ferner können Gemische dieser Denaturierungsmittel verwendet werden.

Die Konzentration des Denaturierungsmittels ist von der Art des Denaturierungsmittels abhängig und für einen Fachmann ohne weiteres feststellbar. Die Konzentration des Denaturierungsmittels (denaturierende Konzentration) ist dann ausreichend, wenn eine vollständige Solubilisierung des denaturierten schwer löslichen Proteins erreicht werden kann. Für Guanidiniumhydrochlorid liegen diese Konzentrationen üblicherweise bei 3-8 mol/l, vorzugsweise 5 - 7 mol/l. Für Harnstoff liegen die Konzentrationen üblicherweise bei 6-10 mol/l. Unter einer schwach denaturierenden Lösung ist eine Lösung zu verstehen, welche ein Denaturierungsmittel in einer Konzentration enthält, welche die Verknüpfung der korrekten Disulfidbrücken im Protein und damit die Ausbildung der natürlichen Tertiärstruktur des Proteins ermöglicht. Vorzugsweise unterscheiden sich stark und schwach denaturierende Lösungen in der Konzentration um den Faktor 100 oder mehr.

Zur vollständigen Monomerisierung der Inclusion Body Proteine ist es weiterhin vorteilhaft, während der Solubilisierung zusätzlich ein Reduktionsmittel wie Dithiothreitol (DTT), Dithioerythritol (DTE) oder 2-Mercaptoethanol in einer Konzentration von 10-400 mM, besonders bevorzugt in einer Konzentration von 20-100 mM zuzusetzen.

Nach der Solubilisierung wird vorzugsweise gegen eine Lösung, welche ein Denaturierungsmittel in einer denaturierenden Konzentration enthält, dialysiert, um ggf. eingesetztes Reduktionsmittel zu entfernen. Zweckmäßig enthält die Lösung, gegen die dialysiert wird, das Denaturierungsmittel in der gleichen Konzentration wie die Denaturierungslösung.

Die anschließende Naturienmg nach dem erfindungsgemäßen Verfahren wird im neutralen bis alkalischen pH-Bereich durchgeführt, vorzugsweise zwischen pH 7 und 10, besonders bevorzugt im pH-Bereich zwischen 7.5 und 9.5. Als Pufferlösungen sind alle üblichen Puffer geeignet. Vorzugsweise werden dem Fachmann bekannte Puffer, wie z.B. Tris- oder Phosphatpuffer, als Renaturierungspuffer verwendet. Zur Überführung des denaturierten Proteins in Renaturierungspuffer wird das solubilisierte Protein in den Renaturierungspuffer verdünnt oder gegen Renaturierungspuffer dialysiert.Dadurch wird das Denaturierungsmittel ebenfalls in seiner Konzentration so weit verdünnt (schwach denaturierende Lösung), daß eine weitere Denaturierung des Proteins nicht mehr erfolgt. Bereits während der anfänglichen Erniedrigung der Konzentration des Denaturierungsmittels kann ein Naturierungsprozeß erfolgen. Die Bedingungen für die Überführung des Proteins in die nicht oder schwach denaturierende Lösung müssen so gewählt sein, daß das Protein im wesentlichen in Lösung bleibt. Zweckmäßig kann dies durch eine langsame kontinuierliche oder eine schrittweise Verdünnung erfolgen. Dabei ist es bevorzugt, das Denaturierungsmittel so zu verdünnen, daß eine möglichst vollständige Naturierung des Proteins erfolgt, oder das Denaturierungsmittel beispielsweise durch Dialyse nahezu vollständig zu entfernen.

Die Naturierung erfolgt vorzugsweise in Gegenwart von niedermolekularen Hilfsstoffen, welche die Ausbeute bei der Naturierung positiv beeinflussen. Derartige Hilfsstoffe sind beispielsweise in R. Rudolph et al., US Patent 5,593,865 beschrieben. Besonders bevorzugt wird als niedermolekularer Hilfsstoffbei der Naturierung Arginin verwendet, zweckmäßig in einer Konzentration von 0.2 bis 1.5 M.

Die Naturierung nach dem erfindungsgemäßen Verfahren erfolgt vorzugsweise unter Zusatz einer Thiolkomponente in reduzierter und oxidierter Form. Bevorzugte Thiolkomponenten sind z.B. Glutathion in reduzierter (GSH) und oxidierter Form (GSSG), Cysteamin und Cystamin, Cystein und Cystin oder 2-Mercaptoethanol und 2-Hydroxyethyldisulfid. Zusatz dieser Thiolreagentien in reduzierter und oxiderter Form ermöglicht sowohl die Ausbildung von Disulfidbrücken innerhalb der sich faltenden Polypeptidkette während der Naturierung als auch das "Reshuffling" falscher Disulfidbrücken innerhalb oder zwischen den sich faltenden Polypeptidketten (Rudolph et al. 1997, loc. cit.).

Das erfindungsgemäße Verfahren wird zweckmäßig während der Naturierung bei niederen Temperaturen (vorzugsweise ca. 10°C) durchgeführt. Bei dem erfindungsgemäßen Verfahren wird die Renaturierung während einer Dauer von 0.5-5 h, bevorzugt von 1-2 h, durchgeführt.

Zur Verhinderung der Oxidation des Reduktionsmittels durch Luftsauerstoff und zum Schutz freier SH-Gruppen ist es zweckmäßig, einen Komplexbildner, wie EDTA, vorzugsweise in einer Menge von 1-20 mM, besonders bevorzugt um ca. 10 mM zuzusetzen.

Unter der "Aktivität von β-NGF" ist die biologische Aktivität von β-NGF zu verstehen. Biologisch aktiver β-NGF liegt als Dimer vor. Die Aktivität kann bestimmt werden nach dem DRG-Test (Dorsal Root Ganglionassay), R. Levi-Montalcini et al., Cancer Res. 14 (1954) 49 und S. Varon et al., Meth. in Neurochemistry 3 (1972) 203. Dabei wird die Stimulierbarkeit und das Überleben von sensorischen Neuronen aus dissoziierten Dorsalwurzelganglien aus Hühnerembryonen an Hand der Ausbildung von Neuriten untersucht.

Die Prosequenz stellt eine vom reifen Protein getrennte Domäne dar. Zwischen diesen Domänen befindet sich eine exponierte Proteasespaltstelle. Diese Spaltstellen lassen sich spezifisch mit geeigneten Proteasen schneiden, bevorzugt z.B.mit einen Protease, welche eine Substrat-spezifität zur Spaltung nach der Aminosäure Arginin besitzt. Trypsin beispielsweise schneidet nach basischen Aminosäuren wie Lysin oder Arginin. In einem geeigneten Verhältnis von proNGF zu Trypsin wird das korrekt gefaltete, mature Protein nicht von der Protease abgebaut. Denaturierte Proteine und auch Faltungsintermediate hingegen exponieren Sequenzen, die dem Angiff der Protease erliegen. Für die Prozessierung von proNGF werden Proteasen mit Trypsin-ähnlicher Substratspezifität bevorzugt. Diese Proteasen spalten das Protein, ohne den aktiven Teil des Proteinmoleküls abzubauen. Als Trypsin-ähnliche Proteasen kommen verschiedene Serin-Poteasen (z.B. Trypsin selbst oder γ-NGF) in Frage. Bevorzugt wird Trypsin eingesetzt. Für die limitierte Proteolyse wird das Protein in einem Masse-Verhältnis von 1:40 bis 1:2500 (Verhältnis Trypsin : proNGF) eingesetzt, bevorzugt wird ein Bereich von 1:40 bis 1:250. Die Proteolyse wird mit einer Inkubationszeit von 1 min - 24 h, bevorzugt 1 - 60 min bei einer Temperatur von 0°C bis 37°C, bevorzugt 0°C bis 20°C, durchgeführt. Als Puffer werden solche verwendet, die die Aktivität der Protease nicht hemmen. Bevorzugt sind Phosphat- und Tris-Puffer im Konzentrationsbereich von 10-100 mM. Die limitierte Proteolyse wird im Bereich des pH-Optimums der Protease durchgeführt, bevorzugt ist ein Milieu von pH 7-8. Nach Ende der Inkubationszeit wird die Proteolyse gestoppt, endweder durch Zugabe eines spezifischen Inhibitors, bevorzugt 1-5 mM PMSF (Phenylmethylsulfonylfluorid) oder Sojabohnen-Trypsininhibitor, bevorzugt 1 mg auf 0.1-5 mg Trypsin oder durch Erniedrigung des pH-Werts auf pH 2-3 durch Zugabe von Säure, bevorzugt HCl (R. Rudolph et al.: Folding proteins. In: T.E. Creighton (ed.): Protein Function: A Practical Approach. Oxford University Press, pp. 57-99 (1997); R H. Edwards und W. J. Rutter, US Patent 5,683,894).

Die folgenden Beispiele, Publikationen und Abbildungen erläutern die Erfindung, deren Schutzumfang sich aus den Patentansprüchen ergibt, weiter. Die beschriebenen Verfahren sind als Beispiele zu verstehen.
- **Figur 1**: zeigt das proNGF-Plasmidkonstrukt pET11a-proNGF für die Expression von rekombinantem humanen proNGF.
- **Figur 2**: zeigt eine Comassie -Färbung eines SDS-PAGE-Gels (15 %) mit Roh-extrakten des E. coli-Stammes BL21(DE3) pET11a-proNGF/pUBS520 vor bzw nach Induktion sowie einer IB-Präparation (SDS-PAGE nach U. K.
Laemmli, Nature 227, 680 (1970)). U: Rohextrakt vor Induktion, I: Roh-extrakt nach vierstündiger Induktion, P: IB-Pellet, S: löslicher Überstand).
- **Figur 2a**: zeigt den Einfluß des pH-Werts auf die Faltung von rh-pro-NGF bei 10°C in 100 mM Tris/HCl, 1 M L-Arginin, 5 mM GSH, 1 mM GSSG, 5 mM EDTA. Die Proteinkonzentration betrug 50 µg/ml, die Faltungsdauer 3 Stunden. Dargestellt sind die Durchschnittswerte aus zwei Meßreihen.
- **Figur 2b**: stellt den Effekt unterschiedlicher L-Arginin-Konzentrationen auf die Faltung von rh-pro-NGF dar. Es wurde bei einem pH-Wert von 9.5 renaturiert; die übrigen Bedingungen waren identisch mit denen der bei der pH-Variation verwendeten. Dargestellt sind die Durchschnittswerte aus zwei Meßreihen.
- **Figur 2c**: zeigt den Einfluß verschiedener GSH-Konzentrationen auf die Faltung von rh-pro-NGF. Die GSSG-Konzentration betrug 1 mM, die L-Arginin-Konzentration 1 M. Die übrigen Renaturierungsparameter waren identisch mit denen bei der Arginin-Variation verwendeten. Dargestellt sind die Durch-schnittswerte aus zwei Meßreihen.
- **Figur 2d**: zeigt den Einfluß verschiedener GSSG-Konzentrationen auf die Renaturierung von rh-proNGF. Die GSH-Konzentration betrug 5 mM. Die restlichen Faltungsparameter waren identisch mit denen bei der GSH-Variation verwendeten. Dargestellt sind die Durchschnittswerte aus zwei Meßreihen.
- **Figur 2e**: stellt den Einfluß verschiedener GdmCl-Gehalte auf die Ausbeute von nativem rh-pro-NGF dar. Der Gehalt an GSH bzw. GSSG betrug 5 mM bzw. 0.5 mM. Die anderen Renaturierungsbedingungen waren identisch mit denen bei der GSSG-Variation benutzten. Dargestellt sind die Durch-schnittswerte aus zwei Meßreihen.
- **Figur 2f**: zeigt den Effekt unterschiedlicher Proteinkonzentrationen auf die Faltungsausbeute von rh-pro-NGF. Die GdmCl-Konzentration betrug in allen Ansätzen 200 mM. Alle anderen Faltungsparameter waren identisch mit denen bei der GdmCl-Variation verwendeten. Dargestellt ist eine Meßreihe.
- **Figur 3**: zeigt das Elutionsprofil der Reinigung von rh-proNGF mittels Kationen-Austauschchromatographie an Poros 20 HS (Perseptive Biosystems, Säulenvolumen 1.7 ml).
- **Figur 4**: zeigt ein SDS-PAGE-Gel (15 %, Silberfärbung nach M. V. Nesterenko, M. Tilley und S. J. Upton, J. Biochem. Biophys. Methods 28, 239 (1994)) der Reinigung von rh-proNGF an Poros 20 HS (1: Auftrag rh-proNGF-Renaturat, 2: Durchlauf, 3: Fraktion 4 (66 bis 69 ml), 4: Fraktion 5 (69-72 ml), 5: Frakton 6 (72-75 ml), 6: Fraktion 7 (75-78 ml), 7: Fraktion 8 (78-81 ml), 8: Fraktion 9 ( 81-84 ml), 9: Fraktion 10 (84-87 ml)).
- **Figur 5**: zeigt das UV-Spektrum von rh-proNGF.
- **Figur 6**: zeigt ein IEX-HPLC-Elutionsdiagramm von rh-proNGF (Säulenmaterial: Poros 20 HS, 100 mm x 4.6 mm Säule, Fa. Perseptive Biosystems).
- **Figur 7**: zeigt ein RP-HPLC-Elutionsdiagramm von rh-proNGF bei 220 nm (Säule Poros 10 R1; 100 mm × 4.6 mm; Perseptive Biosystems).
- **Figur 8**: zeigt ein SDS-Gel (15 %,Coomassie-Färbung) der limitierten Proteolyse von rh-proNGF mit Trypsin (M: 10 kDa-Marker, 1: rh-proNGF-Standard, 2: rh-β-NGF-Standard, 3: Masseverhältnis Trypsin : rh-proNGF = 1 : 40, 4: 1 : 100, 5: 1 : 250, 6: 1 : 500, 7: 1 : 1000, 8: 1 : 2000, 9: 1 : 2500, 10: Kontrolle ohne Trypsin, mit STI).

- **SEQ ID NO:1 und 2**: zeigen Oligonukleotide zur Konstruktion von pET11a-proNGF.
- **SEQ ID NO:3**: zeigt die Nukleotidsequenz der cDNA von humanem proNGF sowie die Aminosäuresequenz des Translationsprodukts.
- **SEQ ID NO:4**: zeigt die Aminosäuresequenz des Translationsprodukts.

### Beispiel 1

### Klonierung der proNGF-cDNA in einen Escherichia coli-Expressionsvektor

Für die Klonierung des proNGF-Konstrukts wurde das T7-Expressionssystem von Novagen gewählt (F.W. Studier und B.A. Moffatt, J. Mol. Biol. 189, (1986) 113). Die für proNGF codierende DNA-Sequenz steht unter der Kontrolle des starken T7 Transkriptionssignals. Als Wirtsstamm wird E. coli BL21 (DE3) verwendet. Das Chromosom enthält das Gen für die T7 RNAPolymerase. Die Expression dieser RNAPolymerase und damit des rh-proNGFs wird durch IPTG (Isopropyl-β-D-thiogalactosid) induziert.

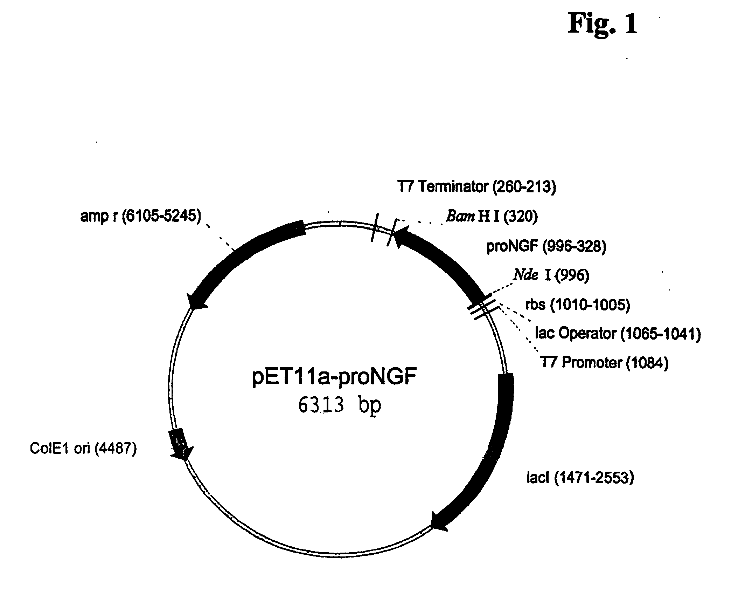

Die cDNA für humanen proNGF wurde durch PCR-Amplifikation aus dem Vektor pMGL-SIG-proNGF von Boehringer Mannheim erhalten (PL-Nr. 1905). Durch Mutageneseprimer wurde am 5'-Ende der für proNGF codierenden DNA-Sequenz eine NdeI- und am 3'-Ende eine BamHI-Schnittstelle eingeführt. Das PCR-Produkt wurde in die NdeI/BamHI-Schnittstelle der multiplen Klonierungsstelle des Vektors pET11a (Novagen) inseriert (Fig. 1).

Es wurden folgende Primer für die PCR benutzt:

### Forward Primer "FwProNGF":

### Reverse Primer "RevNGF":

Nach der Klonierung in den Vektor wurde die Nucleotidsequenz mittels DNA-Sequenzierung überprüft.

### Beispiel 2

### a) Expression von humanem proNGF in E. coli

Für die Anzucht des rekombinanten Bakterienstamms wurde eine Übernachtkultur bereitet. Dazu wurde ein geeignetes Volumen LB-Medium mit 100 µg/ml Ampicillin und 50 µg/ml Kanamycin versetzt.

### LB-Medium (1 l):

10 g Trypton,
10 g Hefeextrakt,
5 g NaCl.

Das Medium wurde mit einer Einzelkolonie beimpft und über Nacht bei 37°C geschüttelt.

Am nächsten Morgen wurde das gewünschte Volumen 2×YT-Medium mit 100 µg/ml Ampicillin und 50 µg/ml Kanamycin im Verhältnis 1:100 (v/v) mit der Übernachtkultur angeimpft. Die Kultur wurde bei 37°C und 200-250 rpm geschüttelt, bis eine OD₆₀₀ von 0.5-0.8 erreicht war. Danach wurde die Expression von proNGF mit 3 mM IPTG 4 h lang bei gleicher Temperatur induziert. Anschließend wurden die Zellen durch Zentrifugation geerntet und entweder sofort aufgeschlossen oder bei -70°C eingefroren.

### 2xYT-Medium (1 I):

17 g Trypton,
10 g Hefeextrakt,
5 g NaCl.

### b) Isolierung der IBs

Das rekombinante Protein fällt in den Bakterienzellen in aggregierter Form an. Die Präparation dieser "Inclusion Bodies" wurde durchgeführt nach R. Rudolph et al.: Folding Proteins. In: T. E. Creighton (ed.): Protein function: A Practical Approach, pp. 57-99.

Für den Zellaufschluß wurden jeweils 5 g Zellpellet in 25 ml 100 mM Tris/HCl pH 7.0; 1 mM EDTA resuspendiert. Danach wurden 1.5 mg Lysozym pro g Zellfeuchtmasse hinzugegeben, 30 min bei 4°C inkubiert und die Zellen anschließend mit dem Gaulin-Zellaufschlußgerät aufgebrochen. Dann wurden 3 mM MgCl₂ sowie 10 µg/ml DNase zum Rohhomogenat gegeben und 30 min bei 25°C inkubiert. Nach dem DNase-Verdau wurden die unlöslichen Zellbestandteile durch Zugabe von 0.5 Volumenanteilen 60 mM EDTA, 6% Triton X-100, 1.5 M NaCl pH 7.0 und anschließende dreißigminütige Inkubation bei 4°C solubilisiert. Die IBs wurden 10 min bei 13.000 rpm abzentrifugiert. Sie wurden dann noch viermal mit je 100 ml 100 mM Tris/HCl pH 7.0; 20 mM EDTA gewaschen und bei -20°C aufbewahrt.

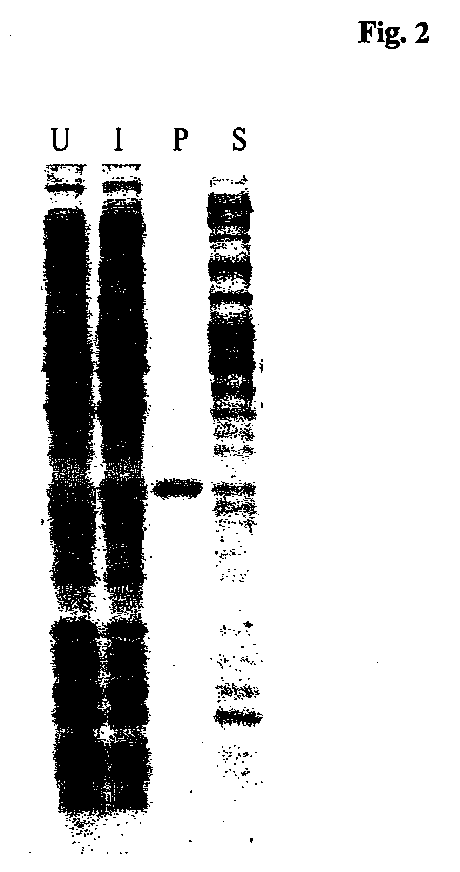

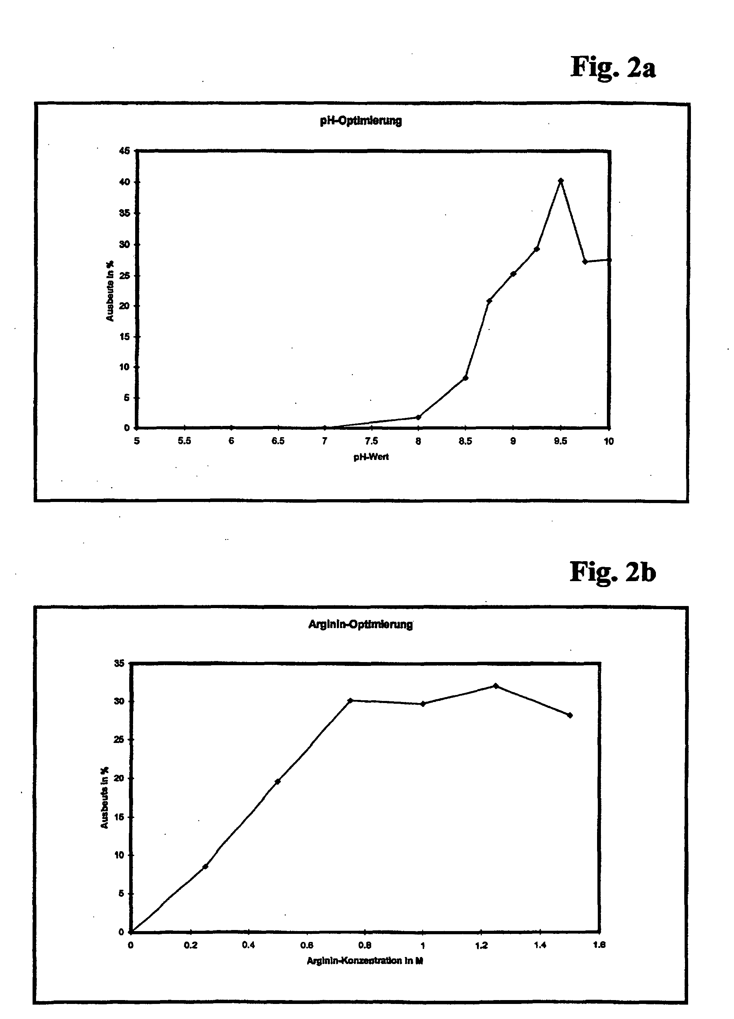

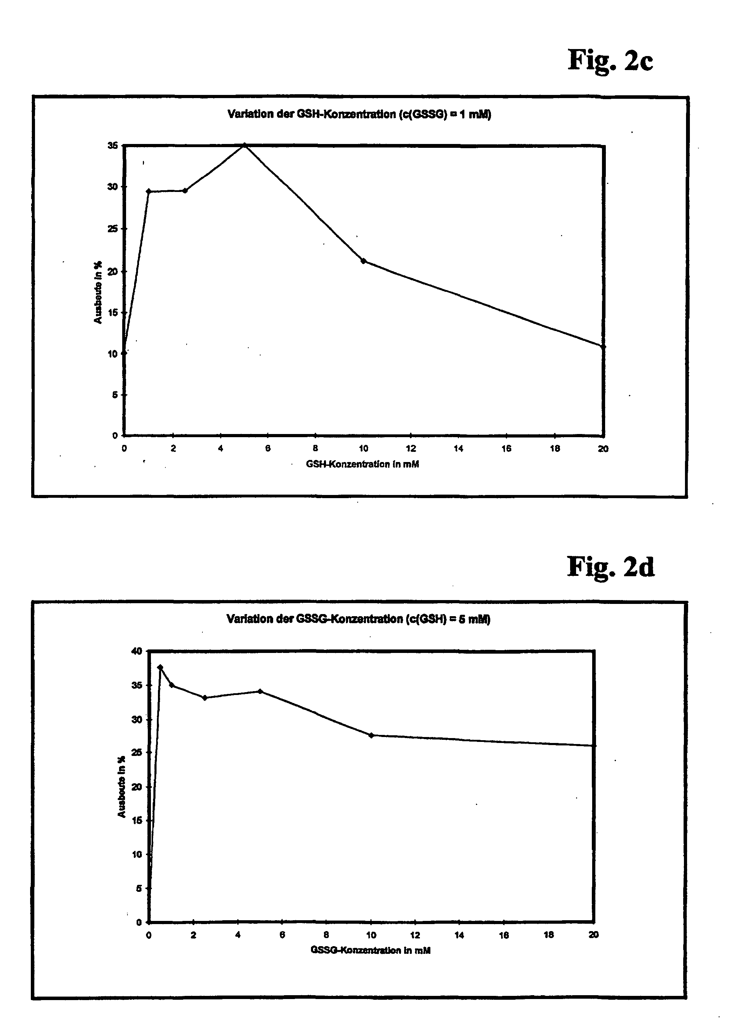

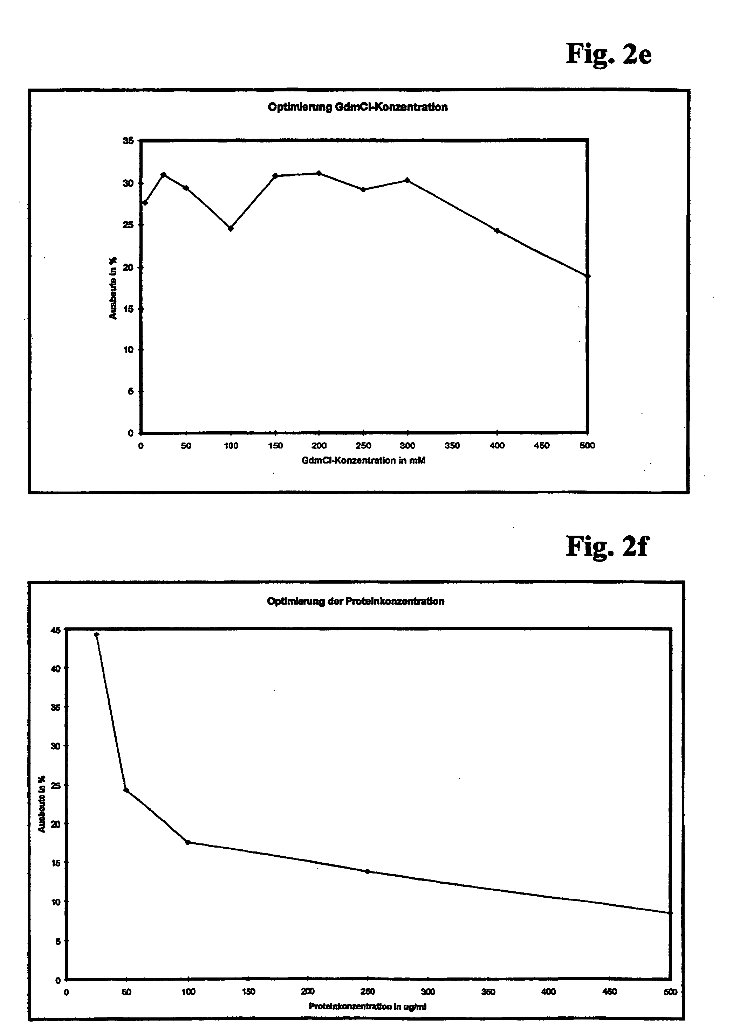

Aus 101 E. coli-Kultur (ca. 44 g Zellfeuchtmasse) wurden auf diese Weise reproduzierbar ca. 4 g IB-Pellet erhalten. Die Präparationen enthielten stets etwa 90-95% rh-proNGF (Fig. 2).

### Beispiel 3

### a) Solubilisierung der IBs

400 mg IB-Pellet wurden in 2 ml Solubilisierungspuffer (100 mM Tris/HCl pH 8.0; 6 M GdmCl; 100 mM DTT; 10 mM EDTA) suspendiert, 2 h bei 25°C inkubiert und 30 min bei 13.000 rpm im Kühlraum abzentrifugiert. Anschließend wurde der Überstand abgenommen und mit 1 M HCl auf pH 3-4 gebracht. Das Solubilisat wurde dreimal gegen 300 ml 6 M GdmCl pH 4.0; 10 mM EDTA dialysiert, und zwar zweimal je 2 h bei 25°C und einmal über Nacht im Kühlraum (12°C; 16-18 h). Die Proteinkonzentration wurde dann nach der Bradford-Methode bestimmt (M. M. Bradford, Anal. Biochem. 72, 248 (1976)). Die Konzentration an rh-proNGF betrug zwischen 40 und 50 mg/ml.

### b) Optimierung der Renaturierung von rh-proNGF

Zur Darstellung von biologisch aktivem rh-pro-NGF aus den in Beispiel 3a) hergestellten Solubilisaten wurden diese in verschiedene Renaturierungspuffer verdünnt. Zur Ermittlung der optimalen Faltungsbedingungen wurden folgende Parameter in der angegebenen Reihenfolge variiert:
a) Temperatur und Zeit,
b) pH-Wert,
c) Arginin-Konzentration,
d) GSH/GSSG-Konzentration,
e) GdmCl-Konzentration,
f) Proteinkonzentration.

Die Ergebnisse sind in Tabellen 1-2 sowieFig. 2a-f dargestellt. Die Menge an renaturiertem rh-proNGF in den Faltungsansätzen wurde mit RP-HPLC bestimmt. Dazu wurden zu bestimmten Zeiten je 925 µl der Faltungsansätze mit 75 µl 32%iger HCl versetzt und dadurch die Faltungsreaktion gestoppt. Für die RP-HPLC-Analytik wurde eine Poros 10 R1-HPLC-Säule und das HPLC-System Beckman Gold mit 125NM Solvent Module, 168 Detektor, Autosampler 507 und Auswertesoftware "Gold V 8.10" verwendet. Die erhaltenen Elutionspeaks wurden mit dem Programm "Peakfit", Version 2.01, gefittet. Zur quantitativen Bestimmung der Ausbeuten wurde eine Eichgerade mit gereinigtem, nativen rh-proNGF erstellt. Da die rh-proNGF-IBs sehr sauber waren, wurde bei der quantitativen Auswertung die zur Renaturierung eingesetzte Gesamtproteinmenge mit der von rh-pro-NGF gleichgesetzt. Bei den dargestellten Meßergebnissen handelt es sich um Durchschnittswerte aus je zwei Messungen.

### Tabelle 1

Optimierung von Temperatur und Zeit bei der rh-pro-NGF-Faltung. Die Proteinkonzentration betrug in jedem Renaturierungsansatz 50 µg/ml. Der Faltungspuffer bestand aus
100 mM Tris/HCl pH 9.5,
1 M L-Arginin,
5 mM GSH,
1 mM GSSG,
5 mM EDTA.

Die Meßreihen wurden mehrfach durchgeführt und mit einer Exponentialfunktion gefittet. Dargestellt sind die Durchschnittsergebnisse zweier Messungen.

| **Temperatur [°C]** | **Endausbeute [%]** | **Plateau erreicht nach ca.** | **Geschwindigkeits- konstante k [s**^{**-1**}**]** |
|---|---|---|---|
| 4 | 25.8 | 3.3 h | 2.596 × 10⁻⁴ s⁻¹ |
| 10 | 29.0 | 1.6 h | 4.865 × 10⁻⁴ s⁻¹ |
| 15 | 22.4 | 1.1 h | 6.399 × 10⁻⁴ s⁻¹ |
| 20 | 12.0 | 1.0h | 1.065 × 10⁻³ s⁻¹ |
| 25 | 11.4 | 0.8 h | 1.935 × 10⁻³ s⁻¹ |

### Tabelle 2

Hier ist der Einfluß verschiedener GSH/GSSG (GSH = reduziertes Glutathion, GSSG = oxidiertes Glutathion) auf die Faltung von rh-pro-NGF dargestellt. Als Renaturierungspuffer wurde
100 mM Tris/HCl pH 9.5,
1 M L-Arginin,
5 mM EDTA
verwendet. Die Faltungsdauer betrug 3 h bei 10°C. In der Tabelle sind die einzelnen Faltungsansätze nach abnehmender Ausbeute geordnet. Angegeben sind die durchschnittlichen Ausbeuten aus zwei Meßreihen.

| **Nr. des Ansatzes** | **GSH/GSSG-Verhältnis [mM]** | **Ausbeute [%]** |
|---|---|---|
| **1** | 5/0.5 | 37.7 |
| **2** | 5/1 | 35.0 |
| **3** | 5/5 | 34.0 |
| **4** | 5/2.5 | 33.1 |
| **5** | 1/1 | 29.4 |
| **6** | 5/10 | 27.6 |
| **7** | 5/20 | 26.0 |
| **8** | 2.5/1 | 22.1 |
| **9** | 10/1 | 21.2 |
| **10** | 1/5 | 18.9 |
| **11** | 20/1 | 10.9 |
| **12** | 0/1 | 9.85 |
| **13** | 0/0 | 0 |
| **14** | 5/0 | 0 |

### c) Renaturierung von rh-proNGF im präparativen Maßstab

Rh-proNGF wurde durch Verdünnung in Faltungspuffer (100 mM Tris/HCl pH 9.5; 1 M L-Arginin; 5 mM GSH; 0.5 mM GSSG; 5 mM EDTA) renaturiert. Dabei wurde bei einer Proteinkonzentration von 50 µg/ml gefaltet. Der Renaturierungsansatz wurde 3 h bei 10°C inkubiert.

### d) Reinigung mittels Ionenaustauschchromatographie

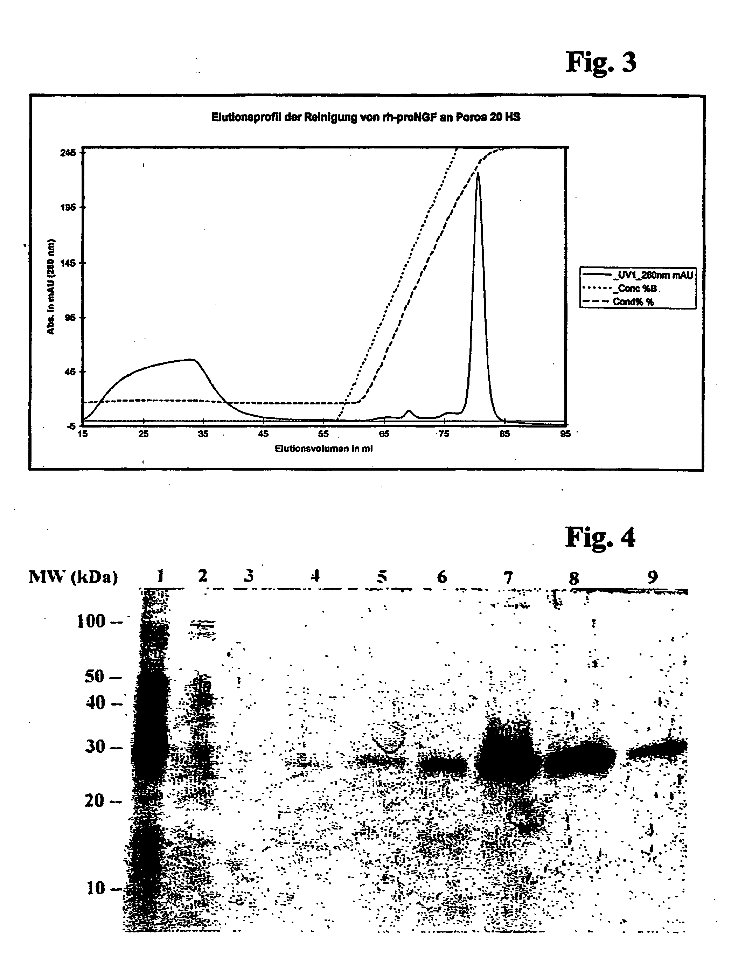

Das Renaturat wurde gegen 10 50 mM Na-Phosphat pH 7.0; 1 mM EDTA (IEX-Puffer A) dialysiert und 30 min bei 20000 rpm abzentrifugiert. Der Überstand wurde auf eine Poros 20 HS-Säule (1.7 ml) aufgetragen und mit einem linearen Salzgradienten eluiert (IEX-Puffer B: 50 mM Na-Phosphat pH 7.0; 1 M NaCl; 1 mM EDTA). Das Protein eluiert bei 980 mM NaCl ( Fig. 3). Nicht nativer rh-proNGF läßt sich nur unter denaturierenden Bedingungen von der Säule entfernen.

### Beispiel 4

### Charakterisierung von rh-proNGF

### a) Konzentrations- und Molekulargewichtsbestimmung mit UV-Spektralphotometrie

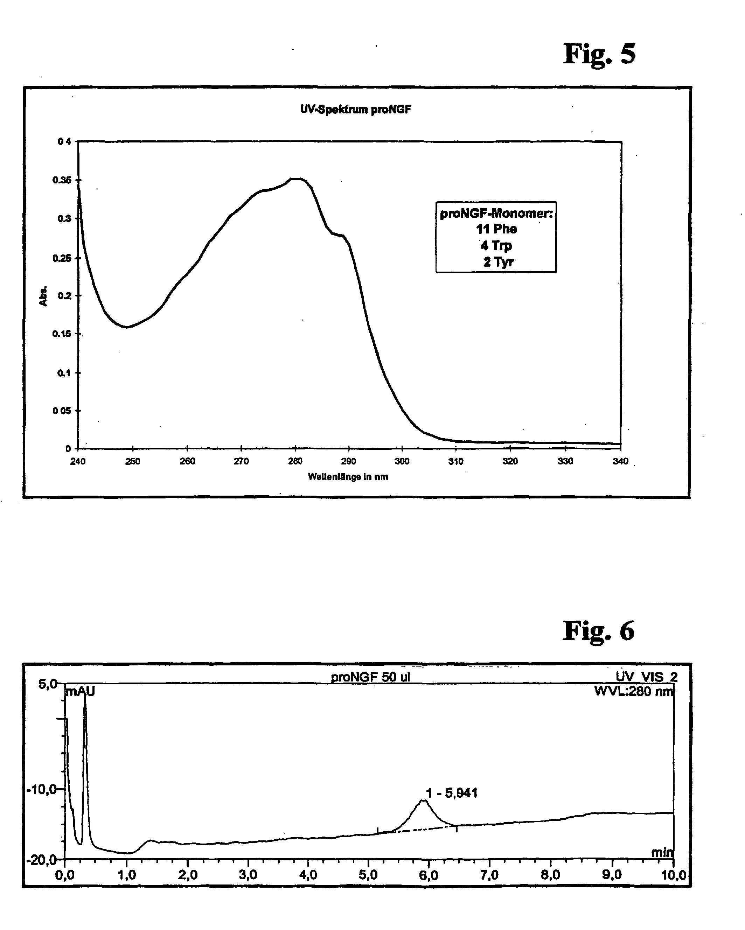

Zur Konzentrationsbestimmung von rh-proNGF in den gereinigten Proben wurde ein UV-Spektrum von 240 bis 340 nm von gegen 50 mM Na-Phosphat pH 7.0; 1 mM EDTA dialysierten Proben gemessen ( Fig. 5; das Spektrum wurde aufgenommen mit einem Beckman DU 640 Spectrophotometer). Die rh-proNGF-Konzentration der Probe wurde aus der Absorption bei 280 nm bestimmt. Für die Berechnung wurde ein theoretischer molarer Extinktionskoeffizient von 256801/(mol×cm) (berechnet nach S. C. Gill und P. H. von Hippel, Anal. Biochem. 182, 319 (1989)) und das Molekulargewicht von 24869 Da pro Monomer (berechnet mit dem ExPASy-Programm "pI/Mw" und korrigiert für drei Disulfidbrücken) zugrundegelegt. Die durch das Spektrum ermittelten Werte stimmten gut mit den durch die Bradford-Methode bestimmten Konzentrationen überein. Die Molekulargewichtsbestimmung erfolgte mittels Elektrospray-Massenspektrometrie. Die theoretische Masse von rekombinantem proNGF beträgt 24869 Da. Experimentell wurden 24871 Da erhalten.

### b) Reinheitsanalyse und Molekulargewiehtsbestimmung mit SDS-Polyacrylamidgelelektrophorese

Es wurden 15%ige Polyacrylamid-Gele verwendet. Die Proben enthielten jeweils 1% (v/v) 2-Mercaptoethanol. Rekombinanter humaner proNGF zeigt im SDS-Gel ein etwas höheres apparentes Molekulargewicht als erwartet: ca. 30 kDa (statt 24.8 kDa) (Fig. 2).

### d) Reinheitsanalyse mit IEX-HPLC

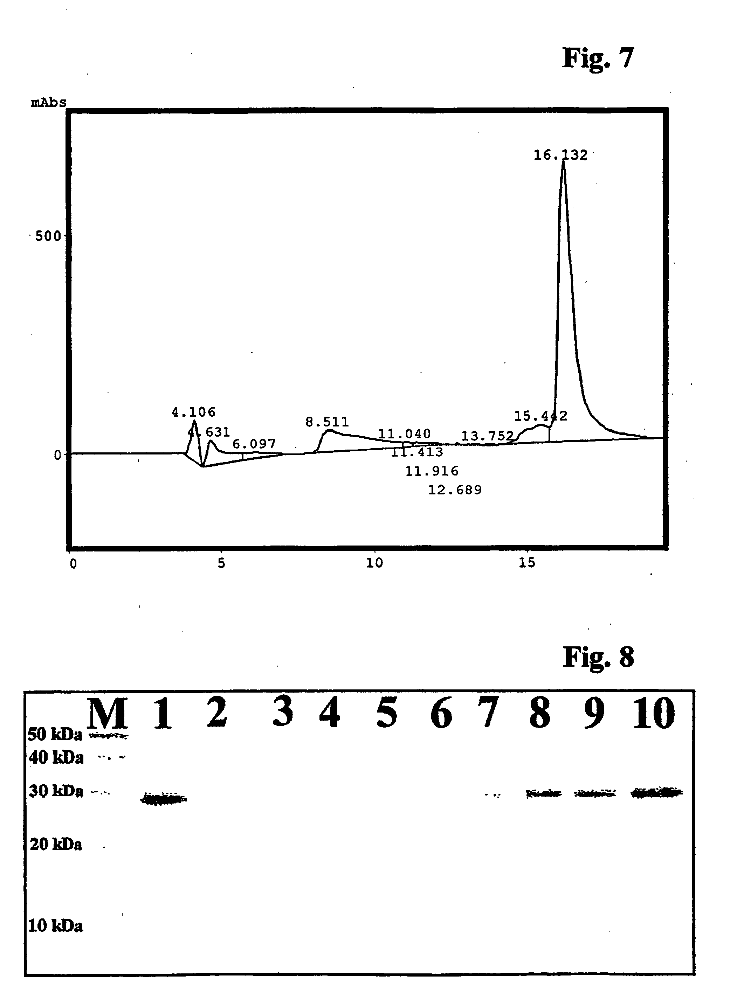

24 µg (50 µl einer Probe mit 0.48 mg/ml rh-proNGF) Protein wurden auf eine mit 50 mM Na-Phosphat pH 7.0; 1 mM EDTA äquilibrierte Poros 20 HS-Säule aufgetragen (125 × 4 mm) und bei einer Flußrate von 5 ml/min mit einem linearen Gradienten von 0 bis 100 % B (B = 50 mM Na-Phosphat pH 7.0; 2 M NaCl; 1 mM EDTA) in 10 Minuten eluiert (Fig. 6). Zur Detektion wurde die Absorption bei 280 nm benutzt (Gyncotek-HPLC-System mit Auswertesoftware Chromeleon Version 3.14).

### e) Reinheitsanalyse mit RP-C4-HPLC

3.1 µg rh-proNGF (15 µl rh-proNGF mit einer Konzentration von 0.21 mg/ml) wurden auf eine mit 0.13% (v/v) TFA äquilibrierte Poros 10 R1-Säule (100 mm × 4.6 mm; Perseptive Biosystems) aufgetragen. Das Protein wurde bei einer Flußrate von 0.8 ml/min mit einem nicht-linearen Gradienten innerhalb von 33 min eluiert (0-4 min: 6%B; 4-9 mm: 6-300%B; 9-24 min: 30-69%B; 24-25 min: 69-100%B; 25-27 min: 100%B; 27- 30 min: 100%B). Als Laufmittel B wurde 0.1% (v/v) TFA in 80% (v/v) Acetonitril verwendet. Zur Detektion wurde die Absorption bei 220 nm herangezogen (Beckman "Gold"-HPLC-System mit Auswertesoftware "Gold V 8.10"). Nativer rh-proNGF eluierte in einem einzigen Peak bei einer Retentionszeit von 14.28 min (Fig.. 7).

### f) N-terminale Sequenzanalyse

Für die N-terminale Sequenzanalyse wurden die mittels RP-HPLC grob gereinigten, solubilisierten IBs verwendet. Die N-terminale Sequenz wurde mit einem Applied Biosystems 476A Protein Sequencer bestimmt. Es wurde folgende Aminosäuresequenz erhalten:

### g) Biologische Aktivität des rekombinanten humanen proNGF

Die physiologische Aktivität von rh-proNGF wurde mit dem DRG-Test (= dorsal root ganglion assay) überprüft (R. Levi-Montalcini, H. Meyer und V. Hamburger, Cancer Res. 14, 49 (1954); S. Varon, J. Nomura, J. R. Perez-Polo und E. M. Shooter, Meth. In Neurochemistry 3, 203 (1972)). Dabei wird die Stimulierbarkeit und das Überleben von sensorischen Neuronen aus dissoziierten Dorsalwurzelganglien von 7-8 Tage alten Hühnerembryonen anhand der Ausbildung von Neuriten untersucht. Die rh- proNGF-Probe wurde mit Kulturmedium auf Konzentrationen von 0.019 bis 20.00 ng/ml eingestellt. Es wurden 15000 Neuronen pro Testansatz eingesetzt. Nach 48-stündiger Inkubation bei 37°C wurde die Zahl der überlebenden Zellen bestimmt. Als Referenz wurde eine Lösung von rh-β-NGF mit bekannter Konzentration hinzugezogen. Bei der quantitativen Auswertung bezieht man sich auf den sogenannten EC50-Wert, d.h. derjenigen NGF-Konzentration, bei der die Hälfte aller Neuronen überleben. Für rh-proNGF ergibt sich ein EC50-Wert von 0.369 ng/ml. Im Vergleich dazu beläuft sich der EC50-Wert für den rh-β-NGF-Standard auf 0.106 ng/ml. Berücksichtigt man die unterschiedlichen Molekulargewichte von rh-β-NGF und rh-proNGF, so ergibt sich für reifen rh-β-NGF eine etwa doppelt so große biologische Aktivität wie für rh-proNGF.

### Beispiel 5

### a) Gewinnung von biologisch aktivem, reifen rh-β-NGF durch limitierte Proteolyse von rh-proNGF

Menschlicher proNGF besitzt als letzte Aminosäure der Prosequenz ein Arginin. Daher kann in vitro aus dieser Vorstufe durch limitierte Proteolyse mit Proteasen geeigneter Substratspezifität wie beispielsweise Trypsin reifer rh-β-NGF erhalten werden. 500 µl gereinigter rh-proNGF wurde gegen 50 mM Tris/HCl pH 8.0 dialysiert. Nach der Dialyse wurde mittels Aufnahme des UV-Spektrums eine Proteinkonzentration von 0.49 mg/ml gemessen. Pro Verdau-Ansatz wurden 20 µg proNGF eingesetzt. Davon wurden nach der Proteolyse 3 µg (entspricht 6 µl) mittels SDS-PAGE analysiert. Als Trypsin-Stammlösungen wurden 0.1 µg/ml bzw. 0.01 µg/ml verwendet. Die Konzentration des Sojabohnen-Trypsin-Inhibitors (STI) betrug 1 mg/ml. Beide Proteine lagen als Lyophilisate vor (Hersteller: Boehringer Mannheim bzw. Sigma) und wurden in obigem Puffer gelöst.

Für die limitierte Proteolyse wurden unterschiedliche Trypsin/rh-proNGF-Masseverhältnisse eingesetzt (s. Tabelle 3). Nach dreißigminütiger Inkubation auf Eis wurde die Reaktion mit je 5 µg STI abgestoppt. Als Kontrolle wurde rh-proNGF ohne Zugabe von Protease ebenfalls auf Eis inkubiert und anschließend mit STI versetzt.

**Tabelle 3**

| **Verhältnis Trypsin:rh-proNGF** | **m(Trypsin) [ µg]** | **V(Trypsin) [µl]** | **V(rh-proNGF) [µl** | **V(STI) [µl]** |
|---|---|---|---|---|
| 1 : 40 | 0.5 | 5 (0.1 µg/ml) | 40 | 5 |
| 1:100 | 0.2 | 2 (0.1 µg/ml) | 40 | 5 |
| 1 : 250 | 0.08 | 0.8 (0.1 µg/ml) | 40 | 5 |
| 1 : 500 | 0.04 | 4 (0.01 µg/ml) | 40 | 5 |
| 1 : 1000 | 0.02 | 2 (0.01 µg/ml) | 40 | 5 |
| 1 : 2000 | 0.01 | 1 (0.01 µg/ml) | 40 | 5 |
| 1 : 2500 | 0.008 | 0.8 (0.01 µg/ml) | 40 | 5 |
| Kontrolle | - | - | 20 | 2.5 |

### b) Analyse der Spaltprodukte durch N-terminale Sequenzierung

Die Verdauansätze mit einem Massenverhältnis Trypsin : rh-proNGF von a) 1 : 40; b) 1: 100 und c) 1 : 250 wurden durch N-terminale Sequenzierung näher untersucht. Bei den beiden oberen Proteinbanden in dem SDS-PAGE-Gel (Abb. 8) handelt es sich um Trypsin (24 kDa) bzw. STI (20.1 kDa). In der Bande bei 13 kDa fanden sich mehrere Spezies:
**N-Terminus 1:** Met⁻¹⁰⁴....;
**N-Terminus 2:** Val⁻³⁵...;
**N-Terminus 3:** Ser¹...(reifer rh-β-NGF);
**N-Terminus 4:** Gly¹⁰... .

Diese Peptide waren in den verschiedenen Ansätzen in unterschiedlichem Ausmaß vorhanden.
Ansatz a): N-Terminus 2 : N-Terminus 3 : N-Terminus 4 = 4 : 5 : 2.
Ansatz b): N-Terminus 2 : N-Terminus 3 = 1 : 1; N-Terminus 4 in Spuren.

Ansatz c) wurde zusätzlich mittels RP-C3-HPLC analysiert (Säule: Nucleosil 500-5 C3-PPN; 125mm x 4 mm). Dabei fanden sich zwei Peaks: Peak 1 (12.32 min): N-Terminus 1; Peak 2 (14.88 min): N-Terminus 2 und N-Terminus 3 im Verhältnis 2 : 3.

Zur Gewinnung von maturem rh-β-NGF aus rh-proNGF im präparativen Maßstab wurden 1.3 mg rh-proNGF (in 50 mM Tris/HCl pH 8.0; Konzentration 0.46 mg/ml) im Masseverhältnis 1:250 (Trypsin : rh-proNGF) mit Trypsin versetzt. Der Ansatz wurde 30 min auf Eis inkubiert. Danach wurde die Protease mit einem 40-fachen Masseüberschuß an Sojabohnen-Trypsininhibitor desaktiviert. Der Spaltansatz wurde gegen 50 mM Natriumphosphat pH 7.0; 1 mM EDTA dialysiert und anschließend auf eine Kationen-Austauschersäule (1.7 ml Poros 20 HS; Perseptive Biosystems) aufgetragen. Das Spaltprodukt eluierte in einem einzigen Peak in einem linearen Salzgradienten von 0 bis 2 M NaCl. Die Elution bei einer Salzkonzentration von etwa 840 mM NaCl entsprach derjenigen von maturem rh-β-NGF in einem Kontrollexperiment. Die Ausbeute an gereinigtem Spaltprodukt betrug 17%.

Die biologische Aktivität des gereinigten Spaltprodukts wurde mittels DRG-Assay getestet. Diese stimmte mit der Aktivität von reifem rh-β-NGF überein (Tab. 4).

**Tabelle 4**

| **Spezies** | **EC50-Wert [pg/ml]** |
|---|---|
| rh-β-NGF | 110 |
| rh-β-NGF, hergestellt durch limitierte Proteolyse von rh-proNGF | 171 |

### Referenzliste

Barnett, J. et al., J. Neurochem. 57 (1991) 1052
Bradford, M. M., Anal. Biochem. 72 (1976) 248
EP-A 0 544 293
Hefti, F. J., J. Neurobiol. 25, (1994) 1418
Hill, S. C. und von Hippel, P., Anal. Biochem. 182 (1989) 319
Laemmli, U. K., Nature 227 (1970) 680
Levi-Montalcini, R., et al., Cancer Res. 14 (1954) 49
Levi-Montalcini, R., Science 237 (1987) 1154
Marston, F.A.., Biochem. J. 240 (1986) 1-12
Nesterenko, M. V. et al, J. Biochem. Biophys. Methods 28 (1994) 239
Rudolph. R. et al. (1997): Folding Proteins. In: T. E. Creighton (ed.): Protein function: A Practical Approach, pp. 57-99
Schmelzer, C. H. et al., J. Neurochem. 59 (1992) 1675
Studier, F.W. et al, J. Mol. Biol. 189 (1986) 113
Suter, U. et al., EMBO J. 10 (1991) 2395
SWISS-PROT Protein Sequence Database No. P01138
Thoenen, H. et al, Physiol. Rev. 60 (1980) 1284
Ullrich, A., et al., Nature 303 (1983) 821
US-Patent 5,235,043
US-Patent 5,593,856
US-Patent 5,606,031
US-Patent 5,683,894
Varon, S. et al., Meth. in Neurochemistry 3 (1972) 203
WO 97/47735
Yankner, B. A., et al., Annu. Rev. Biochem. 51 (1982) 845

## Patentansprüche

1. Verfahren zur Herstellung eines biologisch aktiven β-NGF aus seiner inaktiven schwer löslichen Proform, erhältlich nach rekombinanter Herstellung in Prokaryonten, **dadurch gekennzeichnet, daß** proNGF in seiner inaktiven schwer löslichen Form mit einer Lösung eines Denaturierungsmittels in einer denaturierenden Konzentration gelöst wird, anschließend unter Erhalt der Löslichkeit in eine nicht oder schwach denaturierende Lösung überführt wird und dabei denaturierter proNGF eine biologisch aktive Konformation annimmt, die durch die im natürlichen β-NGF vorliegenden Disulfidbrücken bestimmt ist, und anschließend die Pro-Sequenz abgespalten wird, wobei aktiver β-NGF erhalten wird, der isoliert werden karm, wobei unter proNGF ein beta-NGF zu verstehen ist, ,welcher am N-Terminus mit seiner gesamten Prosequenz verknüpft ist.

2. Verfahren nach Anspruch 1, **dadurch gekennzeichnet, daß** die nicht oder schwach denaturierende Lösung Arginin enthält.

3. Verfahren nach Anspruch 2, **dadurch gekennzeichnet, daß** die Arginin- Konzentration 0,2 bis 1,5 mol/l beträgt.

4. Verfahren nach den Ansprüchen 1 bis 3, **dadurch gekennzeichnet, daß** die Naturierung in Gegenwart einer Thiolkomponente in reduzierter und oxidierter Form erfolgt.

5. Verfahren nach den Ansprüchen 1 bis 4,**dadurch gekennzeichnet, daß** die Abspaltung der Pro-Sequenz durch eine Protease erfolgt, welche eine Substratspezifität zur Spaltung nach der Aminosäure Arginin besitzt.

6. Verfahren nach Anspruch 5, **dadurch gekennzeichnet, daß** als Protease Trypsin verwendet wird.

7. Verfahren nach den Ansprüchen 1 bis 6, **dadurch gekennzeichnet, daß** als Denaturierungsmittel Guanidiniumhydrochlorid oder Harnstoff verwendet wird.

8. Pharmazeutische Zubereitung,
**dadurch gekennzeichnet, daß**
sie rekombinanten proNGF als Wirkstoff zusammen mit üblichen Träger- und Hilfsstoffen enthält, wobei unter proNGF ein beta-NGF zu verstehen ist, welcher am N-Terminus mit seiner gesamten Prosequenz verknüpft ist.

9. Verwendung von rekombinantem proNGF zur Herstellung eines Arzneimittels wobei unter proNGF ein betaNGF zu verstehen ist, welcher am N-Terminus mit seiner gesamten Proseqnenz verknüpft ist.

## Claims

1. A method for the preparation of biologically active β-NGF from its inactive pro form having a poor solubility which is obtainable after recombinant preparation in prokaryotes, wherein proNGF in its inactive form having poor solubility is solubilized in a solution of a denaturing agent in a denaturing concentration and is afterwards transferred into a solution which is not or weakly denaturing whereby solubility is maintained and the denatured proNGF assumes a biologically active conformation as determined by the disulfide bonds present in native β-NGF, and subsequently the prosequence is cleaved off whereby active β-NGF is obtained which can be isolated, wherein proNGF is a beta-NGF which is combined at its N-terminus with its complete pro-sequence.

2. A method according to claim 1 wherein the not or weakly denaturing solution contains arginine.

3. A method according to claim 2 wherein the concentration of arginine is 0.2 to 1.5 mol/l.

4. A method according to any of the claims 1 to 3 wherein the naturation is carried out in the presence of a thiol component in its reduced and oxidized form.

5. A method according to any of the claims 1 to 4 wherein the cleaving off of the prosequence is carried out by means of a protease with a substrate specificity for cleaving after the amino acid arginine.

6. A method according to claim 5 wherein trypsin is used as the protease.

7. A method according to any of the claims 1 to 6 wherein guanidinium hydrochloride or urea is used as the denaturing agent.

8. A pharmaceutical preparation containing recombinant proNGF as the active ingredient together with usual carrier and auxiliary substances, wherein proNGF is a beta-NGF which is combined at its N-terminus with its complete pro-sequence.

9. The use of recombinant proNGF for the preparation of a pharmaceutical preparation wherein proNGF is a beta-NGF which is combined at its N-terminus with its complete pro-sequence.

## Revendications

1. Procédé de fabrication d'un β-NGF biologiquement actif à partir de sa pro-forme inactive difficilement soluble, pouvant être obtenue après fabrication recombinante dans des procaryotes , **caractérisé en ce que** le proNGF sous sa forme inactive difficilement soluble est dissous au moyen d'une solution d'un milieu de dénaturation en une concentration dénaturante, est ultérieurement transformé jusqu'à obtention de la solubilité dans une solution non ou faiblement dénaturante et **en ce que** de la sorte le proNGF dénaturé prend une conformation biologiquement active qui est définie par les ponts disulfures présents dans le β-NGF naturel, et ultérieurement la pro-séquence est détachée par clivage, de manière à obtenir le β-NGF actif , qui peut être isolé, dans lequel par proNGF on entend un β-NGF qui est combiné à l'extrémité N avec sa proséquence tout entière.

2. Procédé selon la revendication 1, **caractérisé en ce que** la solution non ou faiblement dénaturante contient de l'arginine.

3. Procédé selon la revendication 2 , **caractérisé en ce que** la concentration d'arginine s'élève de 0,2 à 1,5 mole/l.

4. Procédé selon les revendications 1 à 3 , **caractérisé en ce que** la re-naturation se passe en présence d'un composant thiol sous forme réduite et oxydée.

5. Procédé selon les revendications 1 à 4 , **caractérisé en ce que** le détachement de la pro-séquence par clivage est effectué au moyen d'une protéase qui possède une spécificité de substrat pour le détachement par clivage après l'amino-acide arginine.

6. Procédé selon la revendication 5 , **caractérisé en ce que** de la trypsine est utilisée en tant que protéase.

7. Procédé selon les revendications 1 à 6, **caractérisé en ce que** du chlorhydrate de guanidinium ou de l'urée est utilisé en tant que moyen de dénaturation.

8. Composition pharmaceutique , **caractérisée en ce qu'**elle comprend du proNGF recombinant en tant que principe actif en mélange avec des supports et auxiliaires usuels, dans laquelle par proNGF on entend un β-NGF qui est combiné à l'extrémité N avec sa proséquence tout entière.

9. Utilisation de proNGF recombinant pour la fabrication d'un médicament, dans laquelle par proNGF on entend un β-NGF qui est combiné à l'extrémité N avec sa proséquence tout entière.


### SEQUENZPROTOKOLL

(1) ALLGEMEINE ANGABEN:
   (i) ANMELDER:
      (A) NAME: BOEHRINGER MANNHEIM GMBH
      (B) STRASSE: Sandhofer Str. 116
      (C) ORT: Mannheim
      (E) LAND: Germany
      (F) POSTLEITZAHL: D-68305
      (G) TELEFON: 08856/60-3446
      (H) TELEFAX: 08856/60-3451
   (ii) BEZEICHNUNG DER ERFINDUNG: Verfahren zur Gewinnung von aktivem beta-NGF
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30B (EPA)
(2) ANGABEN ZU SEQ ID NO: 1:
   (i) SEQUENZKENNZEICHEN:
      (A) LÄNGE: 32 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Forward Primer - FwProNGF"
   (xi) SEQUENZBESCHREIBUNG: SEQ ID NO: 1:
   CGGAATTCCA TATGGAACCA CACTCAGAGA GC 32
(2) ANGABEN ZU SEQ ID NO: 2:
   (i) SEQUENZKENNZEICHEN:
      (A) LÄNGE: 32 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "Reverse Primer - RevNGF"
   (xi) SEQUENZBESCHREIBUNG: SEQ ID NO: 2:
   CCGGATCCTT ATCATCTCAC AGCCTTTCTA GA                                32
(2) ANGABEN ZU SEQ ID NO: 3:
   (i) SEQUENZKENNZEICHEN:
      (A) LÄNGE: 672 Basenpaare
      (B) ART: Nucleotid
      (C) STRANGFORM: Doppel Strang
      (D) TOPOLOGIE: linear
   (ii) ART DES MOLEKÜLS: cDNA
   ( ix) MERKMAL:
      (A) NAME/SCHLÜSSEL: CDS
      (B) LAGE:1..672
   (xi) SEQUENZBESCHREIBUNG: SEQ ID NO: 3:
   ATG GAA CCA CAC TCA GAG AGC AAT GTC CCT GCA GGA CAC ACC ATC CCC      48
   Met Glu Pro His Ser Glu Ser Asn Val Pro Ala Gly His Thr Ile Pro
     1                                                          15
   CAA GTC CAC TGG ACT AAA CTT CAG CAT TCC CTT GAC ACT GCC CTT CGC      96
   Gln Val His Trp Thr Lys Leu Gln His Ser Leu Asp Thr Ala Leu Arg
                    20                  25                  30
   AGA GCC CGC AGC GCC CCG GCA GCG GCG ATA GCT GCA CGC GTG GCG GGG     144
   Arg Ala Arg Ser Ala Pro Ala Ala Ala Ile Ala Ala Arg Val Ala Gly
                35                  40                  45
   CAG ACC CGC AAC ATT ACT GTG GAC CCC AGG CTG TTT AAA AAG CGG CGA     192
   Gln Thr Arg Asn Ile Thr Val Asp Pro Arg Leu Phe Lys Lys Arg Arg
        50                  55                  60
   CTC CGT TCA CCC CGT GTG CTG TTT AGC ACC CAG CCT CCC CGT GAA GCT     240
   Leu Arg Ser Pro Arg Val Leu Phe Ser Thr Gln Pro Pro Arg Glu Ala
    65                  70                  75                      80
   GCA GAC ACT CAG GAT CTG GAC TTC GAG GTC GGT GGT GCT GCC CCC TTC     288
   Ala Asp Thr Gln Asp Leu Asp Phe Glu Val Gly Gly Ala Ala Pro Phe
                    85                  90                  95
   AAC AGG ACT CAC AGG AGC AAG CGC TCA TCA TCC CAT CCC ATC TTC CAC     336
   Asn Arg Thr His Arg Ser Lys Arg Ser Ser Ser His Pro Ile Phe His
               100                 105                 110
   AGG GGC GAA TTC TCG GTG TGT GAC AGT GTC AGC GTG TGG GTT GGG GAT     384
   Arg Gly Glu Phe Ser Val Cys Asp Ser Val Ser Val Trp Val Gly Asp
           115                 120                 125
   AAG ACC ACC GCC ACA GAT ATC AAG GGC AAG GAG GTG ATG GTG TTG GGA        432
   Lys Thr Thr Ala Thr Asp Ile Lys Gly Lys Glu Val Met Val Leu Gly
       130                 135                 140
   GAG GTG AAC ATT AAC AAC AGT GTA TTC AAA CAG TAC TTT TTT GAG ACC        480
   Glu Val Asn Ile Asn Asn Ser Val Phe Lys Gln Tyr Phe Phe Glu Thr
   145                 150                 155                 160
   AAG TGC CGG GAC CCA AAT CCC GTC GAC AGC GGG TGC CGG GGC ATT GAC        528
   Lys Cys Arg Asp Pro Asn Pro Val Asp Ser Gly Cys Arg Gly Ile Asp
                   165                 170                 175
   TCA AAG CAC TGG AAC TCA TAT TGT ACC ACG ACT CAC ACC TTT GTC AAG        576
   Ser Lys His Trp Asn Ser Tyr Cys Thr Thr Thr His Thr Phe Val Lys
               180                 185                 190
   GCG CTG ACC ATG GAT GGC AAG CAG GCT GCC TGG CGG TTT ATC CGG ATA        624
   Ala Leu Thr Met Asp Gly Lys Gln Ala Ala Trp Arg Phe Ile Arg Ile
           195                 200                 205
   GAT ACG GCC TGT GTG TGT GTG CTC TCT AGA AAG GCT GTG AGA TGA TAA        672
   Asp Thr Ala Cys Val Cys Val Leu Ser Arg Lys Ala Val Arg  *   *
       210                 215                 220
(2) ANGABEN ZU SEQ ID NO: 4:
   (i) SEQUENZKENNZEICHEN:
      (A) LÄNGE: 224 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 4:
   Met Glu Pro His Ser Glu Ser Asn Val Pro Ala Gly His Thr Ile Pro
    1               5                  10                  15
   Gln Val His Trp Thr Lys Leu Gln His Ser Leu Asp Thr Ala Leu Arg
               20                  25                  30
   Arg Ala Arg Ser Ala Pro Ala Ala Ala Ile Ala Ala Arg Val Ala Gly
           35                  40                  45
   Gln Thr Arg Asn Ile Thr Val Asp Pro Arg Leu Phe Lys Lys Arg Arg
       50                  55                  60
   Leu Arg Ser Pro Arg Val Leu Phe Ser Thr Gln Pro Pro Arg Glu Ala
   65                  70                  75                  80
   Ala Asp Thr Gln Asp Leu Asp Phe Glu Val Gly Gly Ala Ala Pro Phe
                   85                  90                  95
   Asn Arg Thr His Arg Ser Lys Arg Ser Ser Ser His Pro Ile Phe His
               100                 105                 110
   Arg Gly Glu Phe Ser Val Cys Asp Ser Val Ser Val Trp Val Gly Asp
           115                 120                 125
   Lys Thr Thr Ala Thr Asp Ile Lys Gly Lys Glu Val Met Val Leu Gly
       130                 135                 140
   Glu Val Asn Ile Asn Asn Ser Val Phe Lys Gln Tyr Phe Phe Glu Thr
   145                 150                 155                 160
   Lys Cys Arg Asp Pro Asn Pro Val Asp Ser Gly Cys Arg Gly Ile Asp
                   165                 170                 175
   Ser Lys His Trp Asn Ser Tyr Cys Thr Thr Thr His Thr Phe Val Lys
               180                 185                 190
   Ala Leu Thr Met Asp Gly Lys Gln Ala Ala Trp Arg Phe Ile Arg Ile
           195                 200                 205
   Asp Thr Ala Cys Val Cys Val Leu Ser Arg Lys Ala Val Arg  *   *
       210                 215                 220